Europäisches Patentamt

European Patent Office

Office européen des brevets

(19)

(11) Publication number: **0 268 068**
**A2**

# (12) EUROPEAN PATENT APPLICATION

(21) Application number: **87114886.2**

(22) Date of filing: **13.10.87**

(51) Int. Cl.4 **A61M 29/02**

(30) Priority: **23.10.86 EP 86114736**

(43) Date of publication of application:
**25.05.88 Bulletin 88/21**

(84) Designated Contracting States:
**DE FR GB IT**

(71) Applicant: **KONTRON-HOLDING AG**
**Bernerstrasse Süd 169**
**CH-8010 Zürich(CH)**

(72) Inventor: **Marangoni, Daniele**
**Via Fantoli 16/16**
**I-20138 Milan(IT)**

(74) Representative: **Buntz, Gerhard et al**
**Grenzacherstrasse 124 Postfach 3255**
**CH-4002 Basel(CH)**

(54) **Angioplasty catheter.**

(57) An angioplasty ballon catheter comprises two coaxial lumens and a balloon. The balloon is connected to the distal end and a syringe is connected to the proximal end of the outer lumen. The volume constituted by the balloon, the outer lumen and the syringe is prefilled with a liquid to inflate the balloon.

EP 0 268 068 A2

## Angioplasty Catheter

The invention relates to a balloon catheter for percutaneous angioplasty.

Angioplasty is the most reliable technique for dilating or reopening occluded blood vessels. To employ this technique a double lumen catheter with a distensible balloon at its tip is positioned in the stenotic segment of a blood vessel. The elastic balloon is then inflated resulting in compression of the occlusion, thus dilating the lumen of the blood vessel. The inflation of the balloon is preferably effected with a radiopaque solution to observe it under the X ray monitor. For the inflation a relatively high pressure (100 PSI) is applied.

The filling of the balloon demands high expertise of the medical staff. Moreover, it is time consuming because it is necessary to fill and refill the catheter and the balloon several times in order to eliminate any air bubbles in the balloon. Air bubbles in the balloon would constitute a high risk of explosion if the balloon would break.

As an alternative it has been proposed to use a third lumen connected to the distal tip of the balloon in order to push air bubbles out of the balloon during filling. This system is, however, very expensive. It is technologically complicated to obtain three lumen in 2mm diameter.

It is the purpose of the present invention to provide an angioplasty catheter with which the problems mentioned before in connection with the filling of the catheter with the radiopaque liquid are eliminated.

This is achieved according to the invention with an angioplasty balloon device comprising a double lumen catheter, a balloon connected to the distal end of the outer lumen, a syringe connected to the proximal end of the outer lumen, outer lumen balloon and syringe defining a closed volume which is totally prefilled with a liquid.

The volume defined by the outer lumen, the balloon and the syringe is prefilled under vacuum whereby the generation of air bubbles is absolutely safely avoided. The system is filled with sterilized radiopaque liquid.

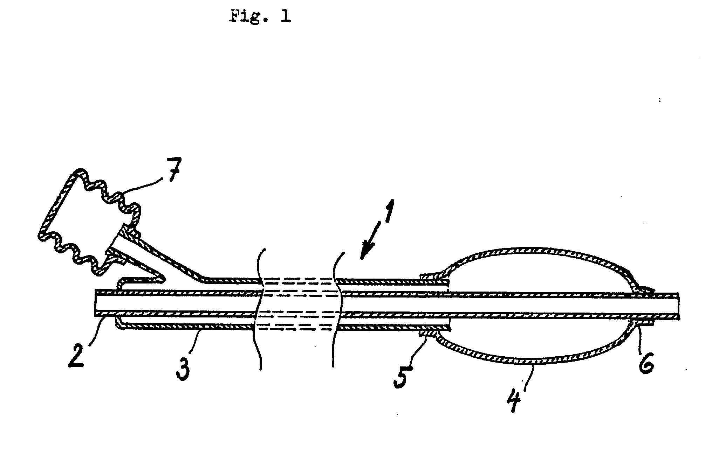

A preferred embodiment of the invention is described in the following by reference to the accompanying drawing.

The drawing shows a conventional catheter 1 of the double lumen type with an inner lumen 2 and an outer lumen 3 arranged co-axially about the inner lumen. A balloon 4 is mounted at the tip of catheter 1. The proximal end 5 of balloon 4 is connected to the open end of the outer lumen. The distal end 6 of balloon 4 is connected to the outside of the inner lumen 2 in an air tight manner.

On the other side of catheter 1 a syringe or pump device is connected to the outer lumen 3. The entire volume of syringe 7, outer lumen 3 and balloon 4 is tilled with a radiopaque liquid.

The filling is effected as a manufacturing step and is performed under vacuum. Thus, it is guaranteed that no air bubbles are present in the volume. The radiopaque liquid is sterile and the entire device is sterilised after manufacture.

When used the device is taken out of its envelope and the catheter is percutaneously introduced in the usual manner. The placement of the balloon can be monitored under X ray. When the balloon has reached the stenotic region and is placed in the occluded part it is simple to apply pressure to the balloon by just compressing the syringe. Due to the guaranteed absence of air bubbles no risk of explosion exists.

A further advantage of the invention resides in the fact that the radiopaque liquid can be kept under a small negative pressure. If during manufacture the ballon 4 is spirally wrapped, the negative pressure will keep it in the wrapped condition until pressure is exerted to inflate the ballon 4.

## Claims

1. Balloon catheter for percutaneous angioplasty comprising a double lumen catheter (1), a balloon (4) connected to the distal end of the outer lumen (3), a syringe (7) connected to the proximal end of the outer lumen, outer lumen, balloon and syringe defining a closed volume which is totally prefilled with a liquid.

2. Balloon catheter according to claim 1, wherein the liquid is radiopaque.

3. Method of manufacturing a balloon catheter according to claim 1 characterized by the fact that the outer lumen (3), balloon (4) and syringe (7) defining closed volume are filled with a liquid under vacuum.

4. Method of manufacturing according to claim 3 characterized by the fact that the balloon (4) is wrapped and the liquid is kept under negative pressure to maintain the balloon in its wrapped condition.

Fig. 1